# EUROPEAN PATENT APPLICATION

(11) **EP 2 754 452 A1**
(43) Date of publication of application: **16.07.2014**
(21) Application number: 13150679.2
(22) Date of filing: 09.01.2013
(51) Int. Cl.: A61K 39/04, C07K 14/35

(54) **SigC for modulation of immune responses**

(71) Applicant: LIONEX Diagnostics and Therapeutics GmbH, 38126 Braunschweig (DE)
(72) Inventor: Singh, Mahavir, 38124 Braunschweig (DE); Oehlmann, Wulf, 30177 Hannover (DE); Spallek, Ralf, 30459 Hannover (DE); Vordermeier, Martin, Woking, Surrey GU24 9NJ (GB); Jones, Gareth, 38126 Braunschweig (DE); Steinbach, Sabine, 38126 Braunschweig (DE)
(74) Representative: Kröncke, Rolf

(57) **Abstract**

The present invention relates in a first aspect to isolated sigma factor C(SigC) of bacteria, in particular, of *mycobacteria* for use in a pharmaceutical composition, e.g., for use in a prophylactic or therapeutic vaccine. The present invention provides the use of SigC of bacteria, in particular, of *mycobacteria* for use in a prime-boost-vaccination strategy, e.g. as a vaccine against *Mycobacterium tuberculosis* and other types of mycobacteria based diseases. In a further aspect, the present invention relates to pharmaceutical compositions containing SigC whereby said pharmaceutical composition is in form of a vaccine. Finally, methods for treating or preventing infections as well as for modulating or reducing innate immune responses are provided based on administration of Sig C.

SEQUENCE LISTING

<110>  Lionex Diagnostics and Therapeutics GmbH

<120>  SigC for modulation of immune responses

<130>  3441_012 EP

<160>  2

<170>  PatentIn version 3.5

<210>  1
<211>  185
<212>  PRT
<213>  M. tuberculosis

<400>  1

Met Thr Ala Thr Ala Ser Asp Asp Glu Ala Val Thr Ala Leu Ala Leu
1               5                   10                  15

Ser Ala Ala Lys Gly Asn Gly Arg Ala Leu Glu Ala Phe Ile Lys Ala
            20                  25                  30

Thr Gln Gln Asp Val Trp Arg Phe Val Ala Tyr Leu Ser Asp Val Gly
        35                  40                  45

Ser Ala Asp Asp Leu Thr Gln Glu Thr Phe Leu Arg Ala Ile Gly Ala
    50                  55                  60

Ile Pro Arg Phe Ser Ala Arg Ser Ser Ala Arg Thr Trp Leu Leu Ala
65                  70                  75                  80

Ile Ala Arg His Val Val Ala Asp His Ile Arg His Val Arg Ser Arg
                85                  90                  95

Pro Arg Thr Thr Arg Gly Ala Arg Pro Glu His Leu Ile Asp Gly Asp
            100                 105                 110

Arg His Ala Arg Gly Phe Glu Asp Leu Val Glu Val Thr Thr Met Ile
        115                 120                 125

Ala Asp Leu Thr Thr Asp Gln Arg Glu Ala Leu Leu Leu Thr Gln Leu
    130                 135                 140

Leu Gly Leu Ser Tyr Ala Asp Ala Ala Ala Val Cys Gly Cys Pro Val
145                 150                 155                 160

Gly Thr Ile Arg Ser Arg Val Ala Arg Ala Arg Asp Ala Leu Leu Ala
                165                 170                 175

Asp Ala Glu Pro Asp Asp Leu Thr Gly
            180                 185

<210>  2
<211>  558
<212>  DNA
<213>  M. Tuberculosis

<400>  2
atgaccgcga cggcaagcga cgacgaggcc gttaccgcac tcgccttgtc ggcggccaag      60

gggaacgggc gggcccttga ggcgtttatc aaagccaccc agcaagacgt gtggcggttc     120

gtcgcctatc tgtccgacgt gggcagtgcg gacgatctca cccaagagac attcctacga     180

gcgatcggcg ccatcccgcg gttttccgca cgctccagcg cccgaacttg gttgctggcc     240

atcgcgcgcc atgtcgtcgc cgatcacatc cgccacgtcc gatcccggcc ccgcaccacc     300

cgcggcgcgc gtcccgaaca tctcatagac ggcgaccgcc atgcccgcgg attcgaagac     360

ctcgtcgagg taaccacgat gatcgccgac ctaaccaccg accaacggga agcgctgctg     420

ctgacccagc tgctcgggct gtcctatgcg gacgccgcgg cggtgtgcgg ctgcccggtg     480

ggcaccatcc gatcccgtgt cgctcgagcg cgcgatgcgc tgcttgccga cgcggagccc     540

gacgacctca ccggctag                                                   558

## Description

The present invention relates in a first aspect to isolated sigma factor C of bacteria, in particular, of *mycobacteria* for use in a pharmaceutical composition, e.g., for use in a prophylactic or therapeutic vaccine. The present invention provides the use of sigma factor C of bacteria, in particular, of *mycobacteria* for use in a prime-boost-vaccination strategy, e.g. as a vaccine against *Mycobacterium tuberculosis* and other types of mycobacteria based diseases. In a further aspect, the present invention relates to pharmaceutical compositions containing sigma factor C whereby said pharmaceutical composition is in form of a vaccine. Finally, methods for treating or preventing infections as well as for modulating or reducing innate immune responses are provided based on administration of Sigma factor C.

### Prior art

The global public health and economic impact of infection with *mycobacterium tuberculosis* and the close related *mycobacterium bovis* is severe. Approximately 9.8 million new cases of *M.tuberculosis* (MTB) infections were estimated for 2010 and recent years have seen a rapid increase in *M.bovis* infections in the UK. To cope the spread of infection, a proved therapeutic, diagnostic and preventative tools are urgently required. For example in cattles, vaccination against *mycobacterium bovis* infection is being considered as one of the long-term policy options for reducing the risk and incidents of bovine TB e.g. in the UK.

Significant progress in developing TB vaccines for cattle has been made over the last seven years. Today strategies against TB, like bovine TB or MTB are envisaged wherein a priming of the immune system is effected with BCG followed by boosting subunit vaccines containing protective agents that are present in BCG or with DNA protein or viral subunit vaccines. These strategies have been shown to give superior protection against experimental challenge in animal models and in cattle. In animals, recent data suggest that neonatal vaccination provides superior protection than BCG vaccination of older animals. However, additional defined subunit vaccine candidates need to be identified to ensure full population coverage. This is in particular true for human TB vaccine development.

A pre-requisite to the selection of new subunit vaccine candidates is that they are recognized not only by infected individuals, but also by T-cells from BCG vaccinated individuals. An additional advantage would be if they would be also recognized by individuals sensitized by environmental *mycobacteria* as this could then provide a further boost of pre-existing immunity by such subunit vaccines. For example, one of the most promising subunit antigen under development in the human and animal field is Ag85A, a ubiquitously expressed antigen across the whole mycobacterial genus.

*Mycobacterium tuberculosis* as a typical example of mycobacteria is a remarkable pathogen capable of adapting and surviving various harsh conditions. Correct gene expression regulation is essential for the success of this process. Transcription initiation is a major step in the regulation of gene expression in procaryotes, like mycobacteria. Although transcription is effected by RNA polymerase, an interchangeable sigma factor is responsible for promoter recognition, thus, directing the whole enzyme to specific genes, the reversible association of different sigma factors is a common mechanism for reprogramming bacterial RNA polymerase and modulating the transcription of numerous genes. For example, 13 putative sigma factors are encoded in the *M.tuberculosis* genome, several being important for virolence. A review of the sigma factors is provided e.g. by Rodrigue, S. et al., FEMS, Microbiol. Ref., 2006, 30, 926-941. Sigma factor C is one of these 13 sigma factors mentioned therein. The nucleic sequence coding for the SigC gene is described in WO 02/18558 identifying the nucleotide sequence coding for SigC gene of coryneform bacteria. Moreover, WO 2006/102767 describes a tuberculosis vaccine and method of making the same wherein the sigma factor kappa is incorporated into the vaccine, thus, providing an improved tuberculosis vaccine. In particular, the sigma factor kappa is introduced into a host organism to be used to produce the tuberculosis vaccine.

Hence, there is still a demand for new vaccines and vaccine strategies allowing improved vaccination against bacterial infections, in particular, mycobacterial infections, like *M.tuberculosis* infections and mycobacteria related thereto. In particular, the object of the present invention is to provide pharmaceutical compositions, in particular, prophylactic or therapeutic vaccines, allowing to boost existing or induced immunity against said bacterial.

### Summary of the invention

The inventors found that isolated sigma factor C of bacteria, in particular, of mycobacteria, is useful in a pharmaceutical composition, in particular for use in a prophylactic or therapeutic vaccine. Sigma factor C (SigC) stimulates and activates innate immunity and is particularly useful in a prime-boost vaccination strategy whereby boosting is possible with administration of SigC only after providing the individual with a specific antigen.

Further, the present invention relates to the use of SigC of bacteria, in particular, of mycobacteria, as a vaccine in combination with other antigens or antigen cocktails used in vaccines, in particular, in combination with mycobacterial antigens, such as Ag85A, Ag85B, or TB10.4

Further, the present invention relates to a pharmaceutical composition containing the SigC whereby SigC is present as active ingredient and/or adjuvant whereby the pharmaceutical composition is adapted to be in form of a vaccine. Said vaccine is particularly useful in preventing or treating infection diseases, cancer, tumors, auto immune diseases, allergies, or chronic or acute inflammatory processes like mycobacterial infection, in particular infection with *Mycobacterium tuberculosis, Mycobacterium bovis, Mycobacterium leprae* or *Mycobacterium ulcerans.*

### Brief description of the drawings

### Figure 1

IFN-γ response of whole blood from naturally *M. bovis* infected cattle, BCG Danish vaccinated cattle and healthy control cattle. Whole blood were stimulated with M. tuberculosis protein SigC (5 µg/ml), PPD-B (10 µg/ml) and ESAT-6-CFP-10 peptide (5 µg/ml) cocktail for 22 - 24 h and the plasma level of IFN-γ determined by ELISA. (A) Results are expressed as a percentage (responder frequency) of animals producing IFN-γ. (B) Box-and -whiskers graph (GraphPad Prism; box extends from the 25^{th} percentile to the 75^{th} percentile, with a line at the median; the whiskers above and below the box show the highest and lowest values) showing the ΔOD₄₅₀ₙₘ values (OD₄₅₀ protein/peptide - OD₄₅₀ medium control) of the individual cattle.

### Figure 2

Cytokine response of whole blood from naturally *M. bovis* infected cattle. Whole blood samples were stimulated with *M. tuberculosis* protein SigC (5 µg/ml) and PPD-B (10 µg/ml) for 22 - 24 h and the plasma concentration from different cytokines determined using MSD technology. Box-and -whiskers graph (GraphPad Prism) showing the stimulation indices (cytokine concentration with protein / cytokine concentration with medium control).

### Figure 3

SigC activates IFN-γ production from natural killer (NK) cells. Whole blood from 5 TB-reactor animals and one uninfected control was cultured in the presence of SigC (5 µg/ml), PPD-B (10 µg/ml), PWM (10 µg/ml) or medium. After 21 hours cells were extracellularly stained with anti-CD3 and anti-CD335 and intracellularly stained using anti-IFN-γ. Stained cells were analysed by flow cytometry. The percentage of IFN-γ producing cell populations is shown. Medium control response subtracted.

### Detailed description of the invention

In a first aspect, the present invention relates to isolated Sigma factor C of bacteria, in particular, of mycobateria for use in a pharmaceutical composition.

As used herein, the term "Sigma factor C" or "SigC" relates to the protein denoted Sigma factor C as described in procaryotes. Included in this definition are variants of the protein showing at least 60 %, e.g. at least 70 %, at least 80 %, preferably at least 90 %, e.g. at least 95 %, or at least 99 % homology to the Sigma factor C amino acid sequences as disclosed in public databases. For example, the Sigma factor C of *M.tuberculosis* H37Rv, denoted RV2069, is shown in Seq. ID No. 1. Seq. ID No. 2 identifies the nucleic acid sequence encoding the polypeptide of Seq. ID No. 1.

The present inventors identified for the first time that SigC is of pharmaceutical and medicinal relevance. In particular, the present inventors noticed that SigC plays an important immunological role. It has been revealed that SigC has adjuvant and vaccine properties in individuals, in particular, in individuals being primed with mycobacteria vaccines, like BCG vaccination or by environmental sensitization against mycobacterial before.

Hence, it is preferred, that SigC of bacteria, in particular of mycobacteria, is used in a prophylactic or therapeutic vaccine. The SigC component may represent an active ingredient but in a preferred embodiment SigC is used an adjuvant in said vaccine.

The present inventors recognized that administration of SigC to an individual activates the innate immune response in said individual. In particular, it has been shown that NK cells are activated after SigC stimulation as determined by IFN-γ release. That is, SigC represents a suitable compound allowing activation of NK cells as member of the innate immunity.

In a preferred embodiment, SigC is derived from mycobacteria, in particular, from MTB. It is preferred that SigC is a polypeptide of Seq. ID No. 1 corresponding to RV2069 derived from the *Mycobacterium tuberculosis* strain HR37Rv. The nucleic acid sequence encoding the polypeptide of Seq. ID No. 1 is shown in Seq. ID No. 2 accordingly. It is particularly preferred, that the SigC according to the present invention is used in a prime-boost-vaccination strategy. The inventors recognized that in pre-sensitized individuals either by previous infection with mycobateria, vaccinated before with BCG or sensitized with environmental mycobacteria components or other environmental components, SigC boost the persisting immunity, in particular, boost the innate immunity of said individual. Moreover, the present inventors recognized that boosting with SigC is sufficient to enhance immunity in said individual.

SigC may be used as the whole protein as exemplified by the polypeptide of Se. ID No. 1 or protein fragments thereof. Unless otherwise indicated, SigC according to the present invention include the whole protein and protein fragments. Preferably, these fragments are at least 8 amino acids long. The skilled person is well aware of suitable methods to determine whether the protein fragments have the same functionality with respect to inducing or enhancing immunity as it is the case for the whole protein. The protein fragment has at least 70 %, preferably 80 %, preferably 90 %, e.g. 95 % or 99 % homology to the natural amino acid sequence as disclosed in databases, like Genebank. Percentage of homology is calculated on the basis of the length of the protein fragment not on the basis of the length of the protein. Thus, a protein fragment with a sequence length of 20 amino acids which includes four amino acids different from the corresponding sequence of the published protein is a 80 % homologue fragment of the published protein.

To determine the homology between amino acid sequences, several computer software packages are known in the art.

The protein may be isolated from natural sources or may be prepared by recombinant technologies or chemical synthesis protocols known to the skilled person. It is clear that the SigC is provided and used in isolated form. Purification of SigC may be performed by recombinant known techniques.

In order to improve properties of SigC, the amino acid sequence may be modified chemically or posttranslationally. For example, the amino acid sequence may consist of both D- and L-amino acids and combinations thereof. These sequences may also comprise modified and/or unusual amino acids. Further, at least one of the amino acids in the respective amino acid sequence may be posttranslationally modified, e.g. by phosphate- or sulphate-modifications, glycosolation, amidation, deaminidation, oxidized or amidated amino acid site chains, etc. Moreover, the component may be modified by PEGylation or other known measures to improve solubility etc.

The terms "Sigma factor C" or "SigC" as used herein encompass the polypeptide itself as well as the fragments, equivalents and homologs thereof, as defined above unless otherwise indicated herein.

The term "vaccination" as used herein means that the object to be vaccinated receives an effective amount of the vaccine to elicit or to enhance an immuno response in order to establish protective immunization.

Vaccination may be obtained by administering the vaccine in advance; that means to elicit a protective immunization, which is also sometimes preferred to as preventive vaccination. Vaccination may also be effected as a therapeutic measure including enhancement of immune response in individuals previously vaccinated or otherwise exposed to the antigen.

As used herein, the term "comprise" or "comprising", or the term "containing" or "containing" and compares the embodiment of "consist of" or "consisting of". That is, the present invention relates preferably to the use of the SigC of bacteria, in particular of mycobacteria in a prime-boost vaccination strategy including priming with specific antigen(s) while boosting the immune response within a suitable time range, typically within a time period of 10 to 14 days after first priming, or in case of previous vaccination with BCG at prenatal age with SigC later. The boost may be effected with SigC alone or in combination with the specific antigen or other adjuvant or antigen respectively. It is preferred, that the boosting with SigC is effected after priming with BCG vaccination or by environmental sensitization against bacteria, in particular, mycobacteria.

It has been recognized that SigC of bacteria, like mycobacteria, according to the present invention is useful in modulating an immune response, in particular, by enhancing TH1 response in the individual boosted therewith accordingly.

It is particularly preferred, that SigC is derived from mycobacteria, like *Mycobacterium tuberculosis, Mycobacterium bovis, Mycobacterium leprae* or *Mycobacterium lucerants* and is used as a vaccine either as protective or therapeutic vaccine against said mycobacteria identified above.

SigC may be administered or used as a vaccine in combination with other antigens derived from the same or different mycobacterial strain or species. For example, SigC may be used in combination with Ag85, PstS1, ESAT6, TB10.4 or other protective antigens derived from mycobacterial pathogens

The present invention provides a pharmaceutical composition containing SigC of bacteria, in particular, of mycobacteria as active ingredient and/or adjuvant in form of a vaccine. Said pharmaceutical composition is particularly useful in preventing or treating infectious diseases, cancer, tumors, auto-immune diseases, allergies, or chronic or acute inflammatory processes.

It is preferred that the pharmaceutical composition is for use in preventing or treating mycobacterial infection, in particular, infection with *Mycobacterium tuberculosis, Mycobacterium bovis, Mycobacterium lepare, Mycobacterium ulcerans.*

The pharmaceutical composition, e.g. in form of a vaccine, according to the invention may also contain pharmaceutically acceptable carriers, adjuvants other than SigC, additional pharmaceutical ingredients, drugs and/or additives. The skilled practitioner will make use of these options in case it seems appropriate.

In addition, the practitioner is well and aware of identifying the suitable dosage of SigC to be administered to said individual. For example, SigC is present in said composition or vaccine at a concentration of from 0.1 to 100 µg/dose

The invention also includes a method of preventing or treating an individual suffering from or being at risk of infection diseases, cancer, tumors, auto-immune diseases, allergies, or chronic or acute inflammatory processes. Said method of treatment comprises the step of administering at least once the Sigma factor C as defined herein in a suitable method to said individual. Prior to the administration of SigC, the method preferably comprises a priming or pre-vaccination of said individual with the specific antigen, e.g. in case of preventing or treating mycobacterial infection, by BCG vaccination or environmental sensitization against mycobacteria. The administration of the SigC may be affected by common means and ways of administration, like systemic or mucosal administration. Typically, priming and boosting is effected by systemic like sub-cutaneous administration of the vaccine. The skilled person is well aware of suitable methods for administration thereof.

The invention will now be described in detail with reference to the figures and examples below without being restricted thereto.

### Methods

**Animals.** All cattle were housed at the Animal Health and Veterinary Laboratories Agency at the time of blood sampling and procedures were conducted within the limits of a United Kingdom Home Office Licence under the Animal (Scientific Procedures) Act 1986, which were approved by the local ethical review committee. Heparinised blood samples were obtained from 13 naturally infected, SICTT-positive reactors from herds known to have bovine tuberculosis (BTB). All TB-reactor animals were subjected to a detailed post-mortem examination and the severity of pathology was scored according to a semi-quantitative system as previously described [17]. In addition, 6 with BCG Danish (SSI, Copenhagen) vaccinated animals (subcutaneous route, 10⁶ CFU) and 9 naïve cows were also tested.

**Bovigam IFN-**γ **release assay (Bovigam IGRA) [19].** Bovigam® ELISA (Prionics AG, Switzerland) was performed according to the manufacturer's instruction. Briefly, heparinised bovine blood was collected, and whole blood cultures were initiated on the day of sampling. Blood cells were stimulated with 5 µg/ml *M. tuberculosis* protein SigC (Lionex GmbH, Braunschweig, Germany) or controls for 20 - 24 h. Controls included: no antigen and 5 µg/ml of bovine purified protein derivative (PPD) (Prionics AG, Switzerland). A peptide cocktail comprising ESAT-6 and CFP-10 derived peptides [20] was also included. Following stimulation, plasma supernatants were harvested and stored at -80 ºC until further processing. IFN-γ levels were quantified using an ELISA-based assay performed with reagents supplied with the kit. Results were scored as positive if the optical density at 450 nm (OD₄₅₀ₙₘ) minus the OD₄₅₀ₙₘ without antigen (ΔOD₄₅₀ₙₘ) was ≥ 0.1 in both of the duplicate wells.

### Bovine 7-plex ELISA [17]

Simultaneous detection of IL-1β, IL-4, IL-6, IL-10, IL-12, IFN-γ and MIP-1β in plasma (generated as described in Bovigam IFN-γ release assay) was performed using electrochemiluminescence based detection methodology of Meso-Scale Discovery (MSD, Gaithersburg, MD, USA (htt ://mesoscale.com/CatalogSystemWeb/WebRoot/index.aspx). Multiplex 96-well plates were supplied with each of the target capture antibodies. All incubation steps were performed at room temperature. Plates were blocked with MSD assay buffer prior to addition of sample or standards. The standards were serially diluted in MSD dilution buffer with top concentration of standard shown in parenthesis: boIL-1β (20 ng/ml bovine IL-1β calibrator, MSD); boIL-4 (2 ng/ml bovine IL-4 calibrator, MSD), boIL-6 (50 U/ml, IAH, Compton, UK), boIL-10 (30 U/ml, IAH), boIL-12 (800 U/ml, IAH), boIFN-γ (50 ng/ml, Endogen, Rock-ford, IL, USA) and huMIP-1β (10 ng/ml, human MIP-1β calibrator, MSD). Following a 2 h incubation, plates were washed and then incubated for a further 2 h with a combined cocktail of directly Sulfotag labelled antibodies (αIL-1β, αIL-4, αIL-6, αIL-10, αIL-12, αMIP-1β (MSD) and biotinylated αIFN-γ together with Streptavidin-Sulfotag (MSD) in MSD dilution buffer. Minimum detectable levels are as follows: IL-1β 0.025 ng/ml , IL-4 3 pg/ml, IL-6 0.05 U/ml, IL-10 0.02 U/ml, IL-12 2 U/ml, IFN-γ 0.050 ng/ml and MIP-1β 2 pg/ml. After a final wash, plates were coated with MSD Buffer-T and luminescence signal measured and analysed on the MSD-6000 Sector Imager.

### Flow cytometric analysis

Whole blood were stimulated with either SigC (5 µg/ml), PPD-B (10 µg/ml), PWM (10 µg/ml) or cell culture medium for 17 hours, following which Brefeldin A was added for a final 5 hours. After lysis of the erythrocytes, whole blood cells were surface stained using CD3:Alexa647 (clone MM1IA, AbDSerotec, MorphoSys UK Ltd, Oxford, UK), CD335:Alexa488 (clone AKS1, AbDSerotec) and Violet fixable dead cell stain (Invitrogen, UK) and then fixed for 16 hours at 4°C in Cytofix (BD Biosciences, UK). Fixed cells were permeabilised in BD Biosciences Permeabilisation Buffer and stained intracellularly with IFN-γ-PE (clone CC302, AbDSerotec). The stained samples were acquired using a CyAn ADP analyzer (Beckman Coulter, USA).

### Results

### Investigating recombinant protein SigC in naïve, BCG vaccinated and M. bovis infected cattle

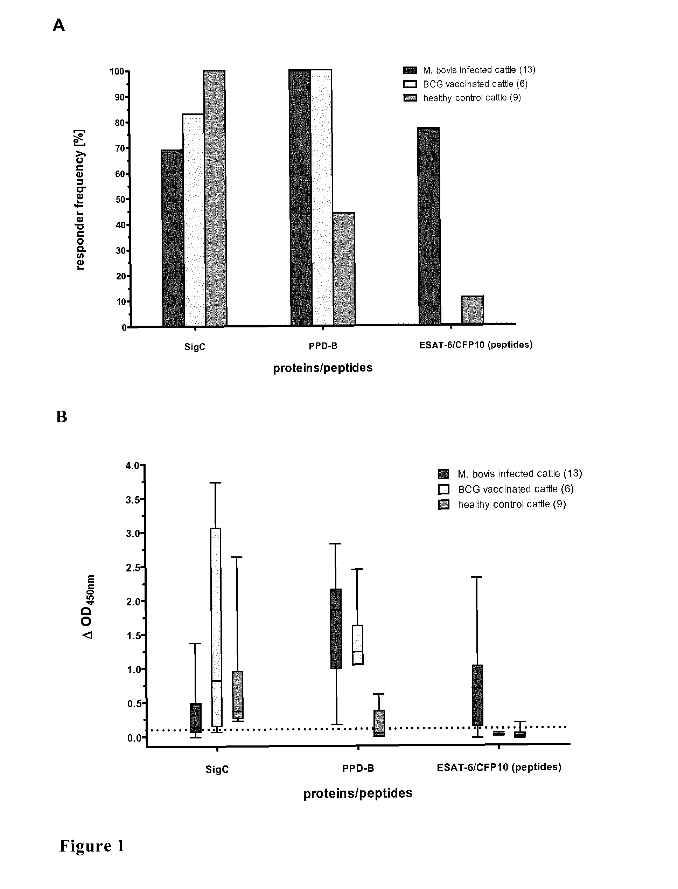

We investigated the IFN-γ response with the recombinant protein SigC using blood from three different categories of cattle, namely *M. bovis* infected cattle (naturally infected with a range of different *M. bovis* isolates), experimentally BCG vaccinated animals as well as uninfected, unvaccinated controls (naïve group). The standard BOVIGAM Interferon-gamma release assay (BOVIGAM IGRA) was used and the results are presented in figure 1. The data demonstrated that SigC was recognised by more than 50 % of *M. bovis* infected animals and by a larger proportion of BCG vaccinated cattle. This is a pre-requisite to be considered as potential subunit vaccine antigen to be used in BCG/subunit heterologous prime-boost protocols.
Interestingly, SigC was also recognised in naïve (healthy) unvaccinated/uninfected cattle (figure 1). However, these healthy animals responded to avian tuberculin PPD (data not shown) suggesting that they were sensitised to one of the ubiquitous environmental *mycobacteria.* Therefore, our working hypothesis is that the T cells recognising this antigen are cross-reactive with homologous proteins expressed by such environmental mycobacterial species. Further characterisation including the mapping of dominant T cell epitopes in combination with *in silico* sequence comparisons will allow us to investigate this hypothesis further.

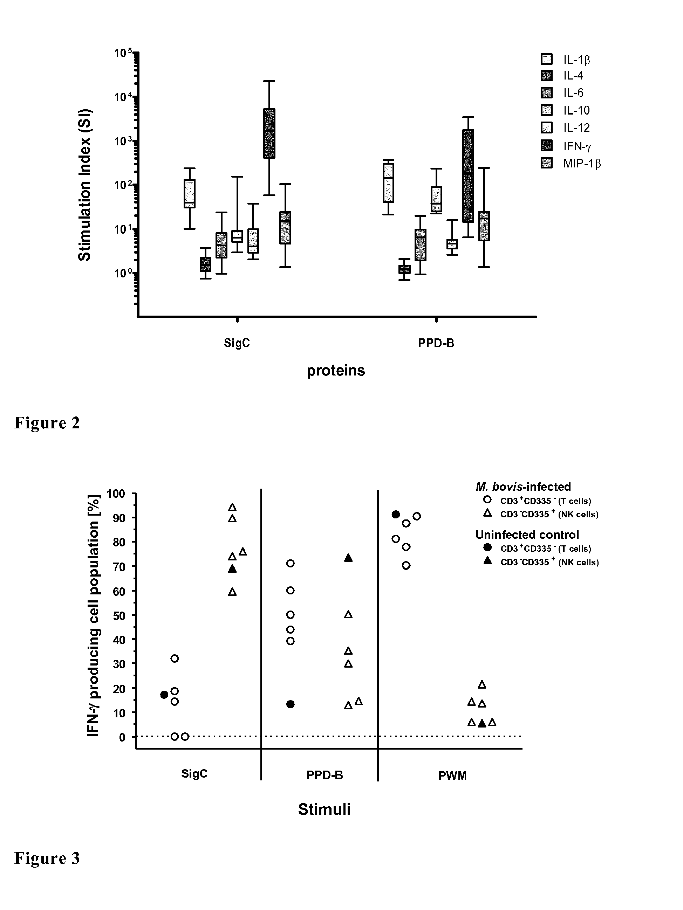

For more information about the kind of immune response induced by SigC we analysed the cytokine profile in whole blood of *M. bovis* infected cattle using a bovine cytokine multiplex. IL-1β, IL-4, IL-6, IL-10, IL-12, IFN-γ and MIP-1β were detected using electrochemiluminescence based detection methodology of Meso-Scale Discovery (Coad M, et al., Vet Res, 41 (2), 14(2010)) (figure 2). As expected, IFN-γ was the dominant cytokine produced by whole blood cells from *M. bovis* infected animals after stimulation with SigC. These results confirm the previous data from the BOVIGAM assay (figure 1) and supports the use of IFN-γ as a readout in primary screening experiments to identify potential diagnostic/vaccine antigens. In addition to IFN-γ, SigC stimulation also resulted in the strong production of IL-1β, whereas all other cytokines were induced to a limited extent. IFN-γ has recently been shown to drive increased IL-1β production in human cells. Thus, in our experiments, SigC induced IFN-γ production may also provide a positive feedback loop resulting in amplification of IL-1β production by blood monocytes.

As detailed above, SigC frequently induced IFN-γ production in whole blood cell cultures from *M. bovis*-infected animals. To investigate the cellular source of this cytokine, whole blood of five *M. bovis*-infected animals (open symbols) and one uninfected control animal (closed symbols) were stimulated with SigC and analysed using flow cytometric analysis (figure 3). In both infected and uninfected animals, the vast majority of cells expressing IFN-γ following stimulation with SigC were CD335⁺ CD3⁻ natural killer cells (triangles). In contrast, stimulation with bovine PPD induced IFN-γ production in a mixed population of cells, including CD3⁺ CD335⁻ T cells (circles) and CD335⁺ CD3⁻ natural killer cells (triangles), while stimulation with the positive control mitogen (PWM) induced IFN-γ production mainly from CD3⁺ CD335⁻ T cells (circles). This data demonstrates that SigC is acting in a major aspect on innate cells and could account for the observation that it induced IFN-γ in all three animal categories studied (*M. bovis* infected, BCG vaccinated, and naïve cattle).

### Discussion:

A stimulation of blood cells isolated from M. bovis infected, BCG vaccinated, or with environmental mycobacteriae sensitised cattle induced frequent and strong IFN-γ responses as well as strong pro-inflammatory cytokine responses as measured by IL-1β (which was measured in infected animals only). The target cell population activated by SigC is almost predominantly composed of NK cells. In addition, SigC is frequently and strongly recognised by blood cells from infected, BCG vaccinated animals as well as animals infected with environmental mycobacterial. Moreover, SigC induced strong cytokine responses associated with TH1 cells, and pro-inflammatory cytokine responses in all three categories of cattle tested (as measured by the production of IFN-γ and IL-1 β)

Thus, SigC is regarded as a potential subunit vaccine antigen capable of boosting immune responses induced by BCG vaccination or environmental sensitisation. As it activates predominantly NK cells, which are known to influence the qualitative outcome of vaccination in respect to T helper subset dominance (e.g. TH1 versus TH2 response), it seems to be an adjuvant component when given in combination with 'conventional' antigens recognised by CD4+ and/or CD8+ T cells. As it also activates cells of the innate immune system in an apparently non-specific manner (NK cells), it seems to be a component of immunotherapy aimed for example at cancer treatment (such as against bladder cancer).

## Claims

1. Isolated sigma factor C (SigC) of bacteria, in particular, of *mycobacteria* for use in a pharmaceutical composition.

2. The SigC of bacteria, in particular, of *mycobacteria,* according claim 1 for use in a prophylactic or therapeutic vaccine.

3. The SigC of bacteria, in particular, of *mycobacteria,* according to claim 1 or 2 for use as an adjuvant in a vaccine.

4. The Sig C of bacteria, in particular, of *mycobacteria,* according to claim 1 for use as an activator of innate immunity.

5. The SigC of bacteria, in particular, of *mycobacteria, for use* according to any one of the preceding claims wherein the sigma factor C is a polypeptide of Seq. ID No. 1.

6. The SigC of bacteria, in particular, of *mycobacteria,* according to any one of the preceding claims for use in a prime-boost vaccination strategy.

7. The SigC of bacteria, in particular, of *mycobacteria, for use* according to claim 6 wherein said prime-boost strategy includes boosting the immune response by SigC after priming with specific antigen(s).

8. The SigC of bacteria, in particular, of *mycobacteria,* for use according to claim 7 wherein priming is effected by BCG vaccination or environmental sensitization.

9. The SigC of bacteria, in particular, of *mycobacteria,* according to any one of the preceding claims for use in modulating an immune response by enhancing Th1 response.

10. The SigC of bacteria, in particular, of *mycobacteria,* for use as a vaccine against *Mycobacterium tuberculosis, Mycobacterium bovis, Mycobacterium leprae or Mycobacterium ulcerans* infection.

11. The SigC of bacteria, in particular, of *mycobacteria,* for use as a vaccine in combination with Ag85, PxtS1, ESAT6, TB10.4 or other protective antigens derived from mycobacterial pathogens.

12. Pharmaceutical composition containing a SigC of bacteria, in particular, of *mycobacteria,* as active ingredient and/or adjuvant in form of a vaccine.

13. Pharmaceutical composition according to claim 12 for use in preventing or treating infectious diseases, cancer, tumors, autoimmune diseases, allergies, or chronic or acute inflammatory processes.

14. Pharmaceutical composition according to claim 12 or 13 for use in preventing or treating mycobacterial infection, in particular, infection with *Mycobacterium tuberculosis, Mycobacterium bovis, Mycobacterium leprae* or *Mycobacterium ulcerans.*
